# EUROPEAN PATENT SPECIFICATION

(11) **EP 3 951 627 B1**
(45) Date of publication and mention of the grant of the patent: **26.06.2024**
(21) Application number: 20779409.0
(22) Date of filing: 14.02.2020
(51) Int. Cl.: G06F 21/32, G06F 21/35, A61B 5/1171

(54) **BIOMETRIC-INFORMATION AUTHENTICATION SYSTEM, BIOMETRIC-INFORMATION AUTHENTICATION METHOD, AUTHENTICATION METHOD, MEASUREMENT INSTRUMENT, MEASUREMENT METHOD, AND COMPUTER-READABLE RECORDING MEDIUM HAVING PROGRAM RECORDED THEREIN**
AUTHENTIFIZIERUNGSSYSTEM FÜR BIOMETRISCHE INFORMATIONEN, AUTHENTIFIZIERUNGSVERFAHREN FÜR BIOMETRISCHE INFORMATIONEN, AUTHENTIFIZIERUNGSVERFAHREN, MESSINSTRUMENT, MESSVERFAHREN UND COMPUTERLESBARES AUFZEICHNUNGSMEDIUM MIT DARAUF AUFGEZEICHNETEM PROGRAMM
SYSTÈME D'AUTHENTIFICATION PAR INFORMATIONS BIOMÉTRIQUES, PROCÉDÉ D'AUTHENTIFICATION PAR INFORMATIONS BIOMÉTRIQUES, PROCÉDÉ D'AUTHENTIFICATION, INSTRUMENT DE MESURE, PROCÉDÉ DE MESURE, ET SUPPORT D'ENREGISTREMENT LISIBLE PAR ORDINATEUR DANS LEQUEL EST ENREGISTRÉ UN PROGRAMME

(30) Priority: 26.03.2019 JP 2019057666
(43) Date of publication of application: 09.02.2022
(73) Proprietor: NEC Corporation, 108-8001 Tokyo (JP)
(72) Inventor: AMANO Katsunori, Tokyo 108-8001 (JP)
(74) Representative: Vossius & Partner Patentanwälte Rechtsanwälte mbB
(86) International application number: PCT/JP2020/005691
(87) International publication number: WO 2020/195300

(56) References cited:
- WO-A1-2017/136940
- WO-A1-2019/049711
- WO-A1-2019/167470
- WO-A2-2015/051253
- JP-A- 2006 155 587
- JP-A- 2017 142 651

## Description

### TECHNICAL FIELD

The present invention relates to a biometric-information authentication system, a biometric-information authentication method, an authentication device, an authentication method, a measurement device, a measurement method, and a computer-readable recording medium having a program recorded thereon.

### BACKGROUND ART

When work for driving a vehicle, an aircraft, a train, a ship, or the like is performed, it is necessary to ensure sufficient rest and sleep in advance. On the other hand, some drivers make a mistake in objectively determining whether or not to start driving work due to overconfidence in their physical strength and some drivers intentionally conceal their lack of sleep and start work, which is an important social issue that causes accidents or the like in relation to safety as well as from the viewpoint of employee health management.

Therefore, a system in which biometric information (an activity amount, a heart rate, a blood pressure, a body temperature, and the like) of a driver is measured by various types of sensors has been proposed to ascertain a health state of the driver or the like. In such a system, technology for performing personal authentication in various schemes has been proposed to authenticate a driver to be measured (hereinafter referred to as a subject).

In Patent Documents 1, 2, and 3, it is recognized that the subject is a specific subject at a timing when biometric information of the subject and alcohol in the subject are detected. Also, in Patent Documents 4 and 5, the subject is requested to input personal identification information at a plurality of timings during measurement of biometric information.

Also, in Patent Document 6, the subject is requested to input personal identification information when it is detected that the subject has stopped exercising, and it is confirmed that the subject himself/herself is wearing a measurement system on the basis of the fact that biometric information changes depending on the rhythm by transmitting rhythm information for prompting the subject to perform a specific exercise to the subject. Also, in Patent Document 7, personal authentication is performed using feature points extracted from an image of the subject.

Also, in Patent Document 8, personal authentication is performed using voiceprint information as a biometric feature of the subject. Also, in Patent Document 9, personal authentication is performed with a pre-registered vein pattern using a wristwatch-type measurement device attached to the subject. Thereby, the subject is authenticated to be a specific subject and validity of data is ensured.

### Prior Art Documents

### Patent Documents

Patent Document 1: Japanese Unexamined Patent Application, First Publication No. 2013-192859
Patent Document 2: Japanese Unexamined Patent Application, First Publication No. 2009-50444
Patent Document 3: Japanese Unexamined Patent Application, First Publication No. 2008-170395
Patent Document 4: PCT International Publication No. WO 2017/209134
Patent Document 5: Japanese Unexamined Patent Application, First Publication No. 2017-213275
Patent Document 6: Japanese Unexamined Patent Application, First Publication No. 2002-238877
Patent Document 7: Japanese Unexamined Patent Application, First Publication No. 2012-10955
Patent Document 8: Japanese Unexamined Patent Application, First Publication No. 2016-49157
Patent Document 9: Japanese Unexamined Patent Application, First Publication No. 2013-212315
WO 2017/136940 A1 and WO 2019/049711 A1 are further related prior art documents.

### SUMMARY

### Problems to be solved by the Invention

However, in Patent Documents 1 to 9, the movement of the subject, the operation of the subject, and the like are required for personal authentication across a plurality of timings when the biometric information is measured or the entire range of a specific period in which biometric information is measured, and thus there is a problem that it is impossible to cope with a situation in which biometric information of a subject is measured for a specific period such as a period in which the subject is sleeping. Also, in Patent Documents 1 to 9, there is a problem that biometric information of a person other than the subject may be measured in a case in which personal authentication is performed only at the beginning of a specific period in which biometric information is measured and for example, the subject transfers a measurement device to a person other than the subject himself/herself after the performing personal authentication.

The present invention has been made in consideration of such circumstances, and an example object of the present invention is to provide a biometric-information authentication system, a biometric-information authentication method, an authentication method, a measurement device, a measurement method, and a computer-readable recording medium having a program recorded therein capable of preventing spoofing.

### Means for Solving the Problems

The present invention is defined by the appended independent claims. Dependent claims constitute embodiments of the invention.

### Example Advantageous Effects of the Invention

According to the present invention, spoofing can be prevented.

### BRIEF DESCRIPTION OF DRAWINGS

FIG. 1 is a conceptual diagram showing a configuration of a biometric-information authentication system 1 according to an example embodiment of the present invention.
FIG. 2A is a functional block diagram showing a configuration of a device A according to the present example embodiment.
FIG. 2B is a functional block diagram showing a configuration of a device B according to the present example embodiment.
FIG. 3 is a flowchart for describing an operation of the device A according to the present example embodiment.
FIG. 4 is a flowchart for describing the operation of the device B according to the present example embodiment.
FIG. 5 is a conceptual diagram for describing an overall operation of the biometric-information authentication system 1 according to the present example embodiment.
FIG. 6A is a conceptual diagram for describing an authentication operation of the biometric-information authentication system 1 according to the present example embodiment.
FIG. 6B is a conceptual diagram for describing an authentication operation according to the background art.
FIG. 7 is a block diagram showing a minimum configuration of an authentication device according to the present example embodiment.
FIG. 8 is a block diagram showing a minimum configuration of a measurement device according to the present example embodiment.

### EXAMPLE EMBODIMENT

Hereinafter, an example embodiment of the present invention will be described with reference to the drawings.

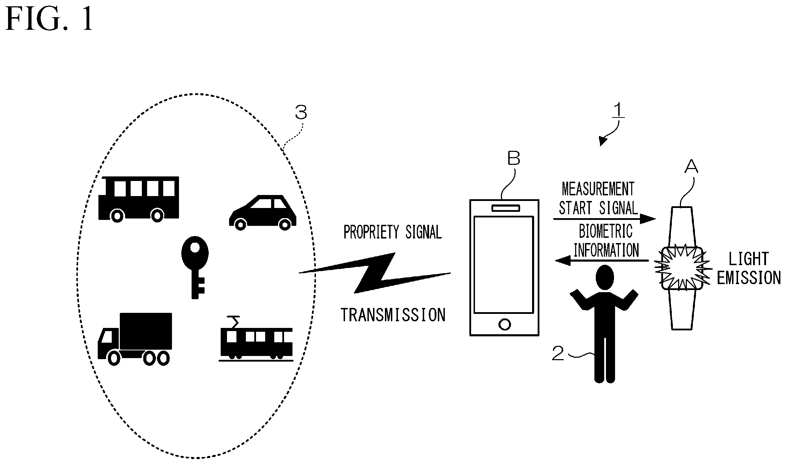

FIG. 1 is a conceptual diagram showing a configuration of a biometric-information authentication system 1 according to the example embodiment of the present invention. In FIG. 1, the biometric-information authentication system 1 includes a device A for measuring biometric information of a subject 2 and a device B equipped with a camera (a photography function). The device A is a wearable terminal worn on a body of the subject 2, and is, for example, an apparatus having a watch-like shape equipped with various types of sensors. Also, the device B is an apparatus possessed by the subject 2, for example, a smartphone. Both the device A and the device B have a communication function and can transmit and receive data in wireless communication.

The device A receives a measurement start signal transmitted in response to a predetermined user operation on the device B. When receiving the measurement start signal, the device A activates a light emission function to emit light in a predetermined light emission pattern. Also, after the completion of personal authentication, the device A measures biometric information of the subject 2 at predetermined time intervals and transmits the measured biometric information to the device B. Also, the device A determines whether or not the device A is being worn on the subject 2 and measures the biometric information of the subject 2 during the device A is being worn on the subject 2; in contrast, if the device A is not being worn on the subject 2, the device A interrupts the measurement of the biometric information of the subject 2 and moves to a process of receiving the above-described measurement start signal and activating the light emission function.

The device B transmits the measurement start signal to the device A in accordance with a predetermined user operation. The device B photographs (the face of) the subject 2 together with the light emission pattern of the device A using the camera (the photography function) before the measurement of the biometric information by the device A. More specifically, the subject 2 wearing the device A on his/her wrist or the like poses so that the face of the subject 2 himself/herself and the light emission state of the device A are within an angle of view in photography by the camera of the device B and photography is performed by the camera of the device B.

The device B authenticates the photographed face of the subject 2 using face recognition technology and determines whether or not the light emission pattern of the device A is a predetermined light emission pattern. If the authentication is successful and the determination is correct, that is, if the photographed face of the subject 2 is a pre-registered face of the subject and the light emission pattern of the device A is the predetermined light emission pattern, the device B determines that the personal authentication has been performed correctly. Also, the device B receives the biometric information measured and transmitted by the device A, determines the propriety of driving of a vehicle 3 or the like by the subject 2 on the basis of, for example, the received biometric information, a change in the biometric information, and/or a history of the biometric information and transmits a propriety signal indicating the propriety of lock opening/closing of the vehicle 3 or the propriety of activation of the vehicle 3.

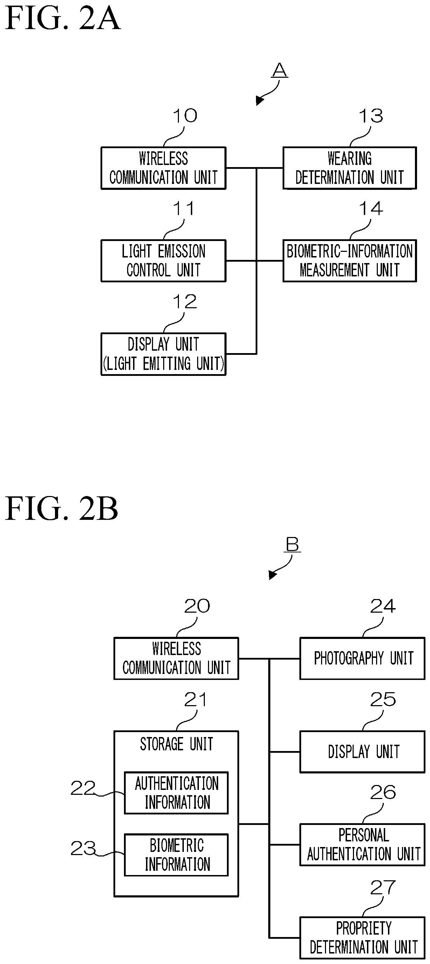

FIG. 2A is a functional block diagram showing a configuration of the device A according to the present example embodiment, and FIG. 2B is a functional block diagram showing a configuration of the device B according to the present example embodiment.

As shown in FIG. 2A, the device A includes a wireless communication unit 10, a light emission control unit 11, a display unit (a light emitting unit) 12, a wearing determination unit 13, and a biometric-information measurement unit 14. The wireless communication unit 10 wirelessly transmits and receives data to and from the device B using WiFi (registered trademark), Bluetooth (registered trademark), or the like. In particular, the wireless communication unit 10 of the present example embodiment performs the reception of the measurement start signal from the device B, the reception of personal authentication OK indicating that the personal authentication has been performed correctly, the transmission of the measured biometric information to the device B, and the like.

When the light emission control unit 11 has received the measurement start signal from the device B, the light emission control unit 11 causes the display unit (the light emitting unit) 12 to be operated so that light is emitted in a predetermined light emission pattern. It should be noted that the predetermined light emission pattern can be designated from the device B or a notification thereof can be provided in advance from the device A to the device B. Also, the light emission pattern and face information of the subject 2 may be associated with each other. By associating the light emission pattern with the face information of the subject 2, the subject 2 and the device A can be associated. The display unit (the light emitting unit) 12 is, for example, a dial/face configured using a liquid crystal or organic electroluminescence (EL), or another light emitting means (a light emitting diode (LED) or the like). The wearing determination unit 13 determines whether or not the device A is being worn on the subject 2 on the basis of a detection result of a predetermined sensor.

The biometric-information measurement unit 14 measures the biometric information (an activity amount, a heart rate, a blood pressure, a body temperature, and the like) of the subject 2 using a predetermined sensor when the personal authentication by the device B is OK. It should be noted that a measurement interval of the biometric information may be a preset time interval or a time interval set at any time by the subject 2. Also, when the wearing determination unit 13 determines that the device A has not been worn on the subject 2, the biometric-information measurement unit 14 interrupts the measurement of the biometric information. The biometric information may be transmitted to the device B by the wireless communication unit 10 each time the measurement is performed, or the biometric information may be saved in a memory (not shown) and the saved biometric information may be collectively transmitted to the device B after the measurement ends. In the description of the operation to be described below, it is assumed that the biometric information is transmitted to the device B each time the measurement is performed.

As shown in FIG. 2B, the device B includes a wireless communication unit 20, a storage unit 21, a photography unit 24, a display unit 25, a personal authentication unit 26, and a propriety determination unit 27. The wireless communication unit 20 uses WiFi, Bluetooth, or the like to wirelessly transmit and receive data to and from the device A and to and from a device (not shown) of the vehicle 3 (a device that controls lock opening/closing and activation of the vehicle). In particular, the wireless communication unit 20 of the present example embodiment performs the transmission of the measurement start signal to the device A, the transmission of the personal authentication OK indicating that the personal authentication has been performed correctly to the device A, the reception of the measured biometric information from the device A, the transmission of a propriety signal indicating the propriety of lock opening/closing or activation of the vehicle to the device (not shown) of the vehicle 3, and the like.

The storage unit 21 is a memory for storing various types of data and stores authentication information 22 and biometric information 23. The authentication information 22 includes face information for identifying and authenticating the subject 2 and a light emission pattern for identifying and authenticating the device A. The biometric information 23 includes the biometric information measured by the device A. The photography unit 24 is a camera provided in the device B and photographs a state in which the subject 2 wearing the device A on his/her wrist or the like poses so that the face of the subject 2 and the light emission state of the display unit (the light emitting unit) 12 of the device A are within an angle of view in photography by the photography unit 24 of the device B when personal authentication is performed before the measurement of biometric information.

The display unit 25 is a display configured using a liquid crystal or organic EL display, or the like, and displays icons, messages, various types of menus, captured images (including moving images), and the like. The personal authentication unit 26 uses the authentication information 22 to authenticate the photographed face of the subject 2 using the face recognition technology, and determines whether or not the light emission pattern of the device A is a predetermined light emission pattern. If the authentication is successful and the determination is correct, that is, if the photographed face of the subject 2 is a pre-registered face of the subject and the light emission pattern of the device A is a predetermined light emission pattern, the personal authentication unit 26 determines that the personal authentication has been performed correctly.

The propriety determination unit 27 determines the propriety of driving of the vehicle 3 or the like by the subject 2 on the basis of, for example, the biometric information 23 measured by the device A, a change in the biometric information 23, and/or a history of the biometric information 23. The propriety signal indicating the propriety determination is transmitted by the wireless communication unit 20 to a device (not shown) of the vehicle 3 (a device that controls lock opening/closing and activation of the vehicle).

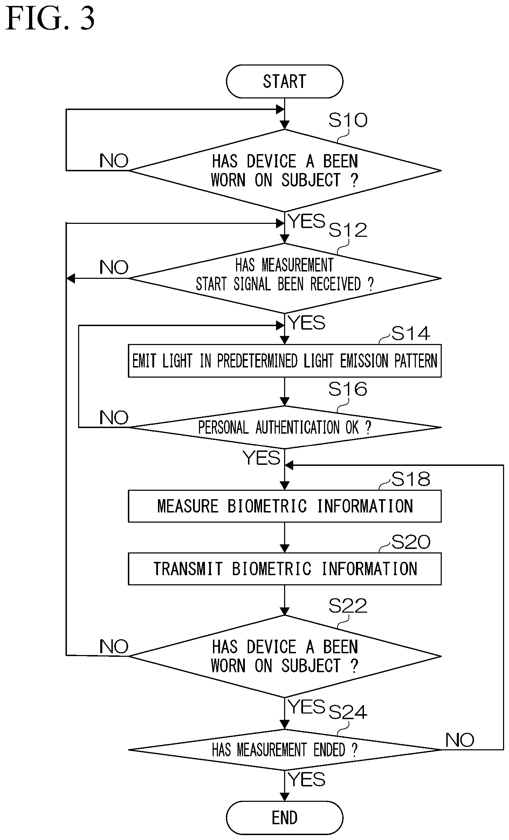

FIG. 3 is a flowchart for describing an operation of the device A according to the present example embodiment. In FIG. 3, the device A determines whether or not the device A is being worn on the subject 2 on the basis of a detection result of the predetermined sensor using the wearing determination unit 13 (step S10). Then, if the device A has not been worn on the subject 2 (NO in step S10), the device A is in a standby state continuously. That is, the measurement of biometric information is not performed in a state in which the device A is not worn on the subject 2. In contrast, if the device A is being worn on the subject 2 (YES in step S 10), the device A determines whether or not a measurement start signal has been received from the device B via the wireless communication unit 10 (step S12). Then, if the device A has not received the measurement start signal (NO in step S12), the device A is in the standby state continuously until the measurement start signal is received.

In contrast, if the device A has received the measurement start signal (YES in step S12), the device A activates the display unit (the light emitting unit) 12 so that light is emitted in a predetermined light emission pattern by the light emission control unit 11 (step S14). At this time, when the subject 2 visually recognizes the light emission of the display unit (the light emitting unit) 12 of the device A, the subject 2 wearing the device A on his/her wrist or the like poses so that the face of the subject 2 himself/herself and the display unit (the light emitting unit) 12 of the device A are within an angle of view in photography by the camera of the device B and performs the photography operation from the device B.

Next, the device A determines whether or not the personal authentication OK indicating that the personal authentication has been performed correctly has been received from the device B (step S16). Then, if the device A has not received the personal authentication OK from the device B (NO in step S 16), the device A returns the process to step S14 and continuously performs light emission based on the predetermined light emission pattern until the personal authentication OK is received. It should be noted that if the device A has not received the personal authentication OK even though a predetermined time period has elapsed, the device A may determine that the personal authentication has not been performed correctly and may return the process to step S12 or end the process shown in FIG. 3.

In contrast, if the device A has received the personal authentication OK from the device B (YES in step S 16), the device A starts the measurement of biometric information (an activity amount, a heart rate, a blood pressure, a body temperature, and the like) of the subject 2. That is, in the device A, the biometric-information measurement unit 14 measures the biometric information of the subject 2 using a predetermined sensor (step S18) and the wireless communication unit 10 transmits the measured biometric information to the device B (step S20). It should be noted that the subject 2 is transferred to a sleeping state after the personal authentication is correctly performed. That is, the measured biometric information indicates a physical state of the subject 2 during sleep.

Next, in the device A, the wearing determination unit 13 determines whether or not the device A is being worn on the subject 2 on the basis of a detection result of the predetermined sensor (step S22). Then, if the device A is being worn on the subject 2 (YES in step S22), the device A determines whether or not the measurement of the biometric information has ended (step S24). It should be noted that a case in which it is determined that the measurement has ended is, for example, a case in which the device A or the device B performs a predetermined end operation, a case in which the device A is not worn again on the subject 2 even though a predetermined time period has elapsed after the device A was removed from the subject 2, a case in which no biometric information is transmitted from the device A even though a predetermined time period has elapsed after the device A was removed from the subject 2. Then, if the measurement of the biometric information has not ended (NO in step S24), the device A returns the process to step S18 and continuously performs the measurement of the biometric information of the subject 2 and the transmission of the biometric information.

In contrast, if the device A has been removed from the subject 2 during the measurement of the biometric information (NO in step S22), the device A interrupts the measurement of the biometric information, returns the process to step S12, and re-executes the personal authentication. It should be noted that when the device A detects that the device A has been removed from the subject 2, the device A may notify the device B that the device A has been removed from the subject 2. Also, when the measurement of the biometric information has ended (YES in step S24), the device A ends the process shown in FIG. 3.

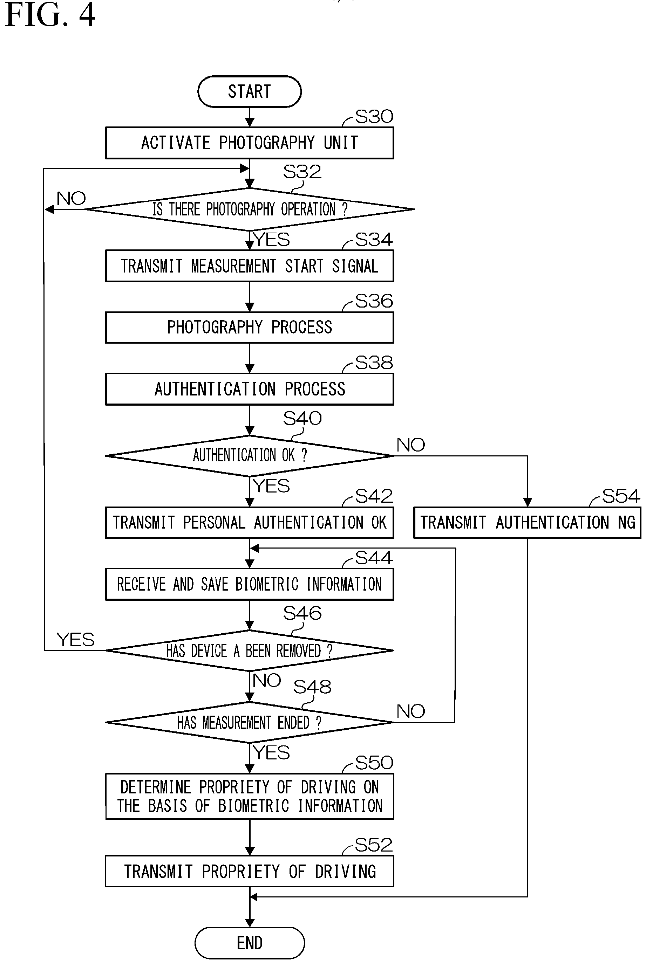

FIG. 4 is a flowchart for describing an operation of the device B according to the present example embodiment. In FIG. 4, the device B activates the photography unit 24 from a predetermined application (step S30). Next, the device B determines whether or not there is a photography operation (an operation on a shutter button displayed on the display unit 25) (step S32). Then, if there is no photography operation (NO in step S32), the device B is in the standby state (displays a live view) continuously.

In contrast, if there is a photography operation (YES in step S32), the device B transmits a measurement start signal to the device A (step S34) and executes a photography process of the photography unit 24 (step S36). At this time, the device A causes the display unit (the light emitting unit) 12 to emit light in a predetermined light emission pattern in response to the measurement start signal that has been received. The photography operation is performed with the device B in a state in which the subject 2 sees the live view displayed on the display unit 25 of the device B and the subject 2 poses so that the face of the subject 2 himself/herself and the display unit (the light emitting unit) 12 of the device A being worn on the wrist or the like of the subject 2 are within an angle of view in photography by the camera of the device B.

After the photography process of the photography unit 24, the personal authentication unit 26 of the device B authenticates the photographed face of the subject 2 using the authentication information 22 of the storage unit 21 with the face recognition technology and determines whether the light emission pattern of the display unit (the light emitting unit) 12 of the device A is a predetermined light emission pattern (step S38). Next, the device B determines whether or not the personal authentication by the personal authentication unit 26 is OK, that is, whether or not the face of the photographed subject 2 is a pre-registered face of the subject and the light emission pattern of the display unit (the light emitting unit) 12 of the device A is a predetermined light emission pattern (step S40). Then, if the personal authentication has been performed correctly (YES in step S40), the device B transmits the personal authentication OK indicating that the personal authentication has been performed correctly to the device A using the wireless communication unit 20 (step S42). The subject 2 is transferred to a sleeping state if personal authentication is performed correctly. If the personal authentication OK is received, the device A starts the measurement of biometric information of the subject 2 and the transmission of the biometric information. That is, the measured biometric information indicates the physical state of the subject 2 during sleep.

Next, the device B receives the biometric information transmitted from the device A via the wireless communication unit 20, and saves the received biometric information in the storage unit 21 (step S44). Next, the device B determines whether or not the device A has been removed from the subject 2 (step S46). The device B may recognize that the device A has been removed from the subject 2 due to the notification from the device A or the interruption of the transmission of the biometric information by the device A. Then, if the device A has been removed from the subject 2 during the measurement of the biometric information by the device A (YES in step S46), the device B returns the process to step S32 and re-executes the personal authentication.

In this way, if the device A is removed during the measurement of biometric information, the personal authentication is re-executed in a state in which the device A is being worn. Thus, it is possible to prevent spoofing in which the device A is transferred to another person to measure false biometric information after personal authentication is performed only when the device A for measuring biometric information is activated. Also, because spoofing can be prevented, it is possible to ensure that the measured biometric information is the biometric information of a specific subject across the entire range of a specific period including a period in which the subject 2 is sleeping. Therefore, personal authentication in which the subject 2 is required to bear a burden or perform a special operation can be executed only for a partial period without being executed across the entire range of the specific period that also includes the period in which the subject 2 is sleeping, and the biometric information of the subject 2 can be measured in a state in which the sleep of the subject 2 is not disturbed.

In contrast, if the device A has not been removed from the subject 2, that is, when a state in which the device A is being worn on the subject 2 continues (NO in step S46), the device B determines whether or not measurement has ended (step S48). It should be noted that a case in which it is determined that the measurement has ended is, for example, a case in which a predetermined end operation has been performed on the device A or the device B, a case in which a process of the device A does not transition to a light emission operation even though a predetermined time period has elapsed after the device A was removed from the subject 2. Then, if the measurement of the biometric information has not ended (NO in step S48), the device B returns the process to step S44 and continues the reception and saving of the biometric information of the subject 2.

In contrast, if the measurement of the biometric information has ended (YES in step S48), the propriety determination unit 27 of the device B determines the propriety of driving of the vehicle 3 or the like by the subject 2 on the basis of, for example, the biometric information 23 received from the device A and saved in the storage unit 21, a change in the biometric information 23, and/or a history of the biometric information 23 (step S50). In the present example embodiment, because the measured biometric information indicates the physical state of the subject 2 during sleep, the propriety determination unit 27 determines whether or not the subject 2 has obtained sufficient sleep, that is, determines the propriety of driving of the vehicle 3 or the like by the subject 2, on the basis of the biometric information (an activity amount, a heart rate, a blood pressure, a body temperature, and the like) during sleep. Next, the wireless communication unit 20 of the device B transmits a propriety signal indicating the propriety determination to a device (not shown) of the vehicle 3 (a device that controls lock opening/closing and activation of the vehicle) (step S52). Subsequently, the device B ends the process shown in FIG. 4.

In contrast, if the personal authentication has not been performed correctly (NO in step S40), the wireless communication unit 20 of the device B transmits an authentication NG indicating that the personal authentication has not been performed correctly to the device A and the process shown in FIG. 4 ends. If the authentication NG has been received from the device B, the device A may re-execute light emission in accordance with a predetermined light emission pattern or may end the process shown in FIG. 3.

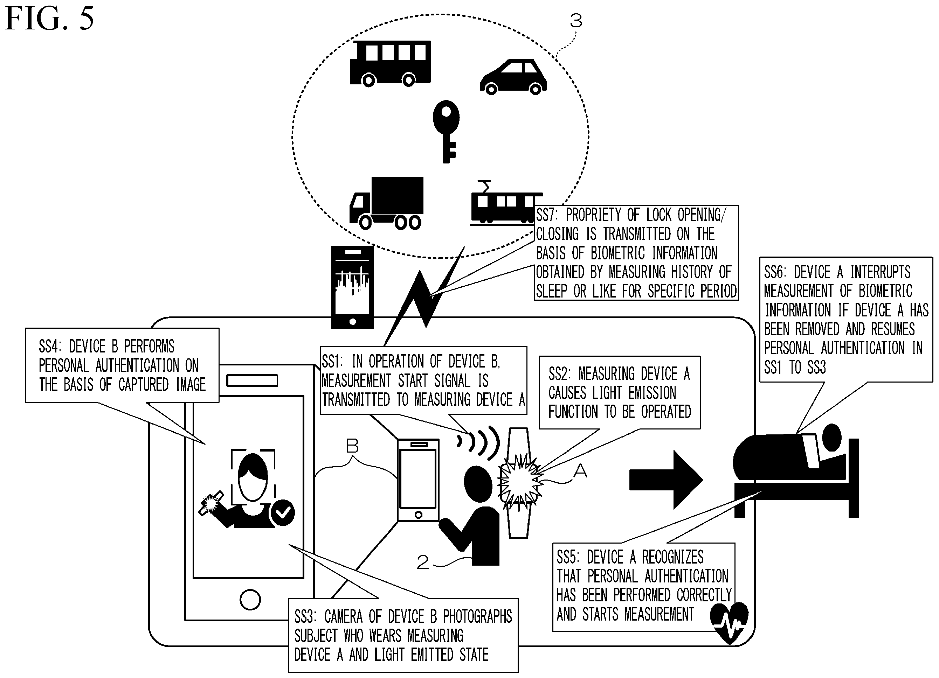

FIG. 5 is a conceptual diagram for describing an overall operation of the biometric-information authentication system 1 according to the present example embodiment. In FIG. 5, first, a photography operation is performed with the device B in a state in which the subject 2 sees a live view displayed on the display unit 25 of the device B and the subject 2 wearing the device A on his/her wrist or the like poses so that the face of the subject 2 himself/herself and the display unit (the light emitting unit) 12 of the device A are within an angle of view in photography by the camera of the device B. When there is a photography operation, the device B transmits a measurement start signal to the device A (step SS1). When receiving the measurement start signal, the device A causes the display unit (the light emitting unit) 12 to emit light in a predetermined light emission pattern (step SS2). The device B photographs a state in which the subject 2 wearing the device A on his/her wrist or the like poses so that the face of the subject 2 and the light emission state of the display unit (the light emitting unit) 12 of the device A are within an angle of view in photography by the photography unit 24 of the device B (step SS3).

The device B performs personal authentication on the basis of a captured image (including a moving image) and determines that the personal authentication has been performed correctly if the light emission pattern of the display unit (the light emitting unit) 12 of the device A is a predetermined light emission pattern and the subject 2 is an authenticated person (step SS4). The device A recognizes that the personal authentication has been performed correctly and starts the measurement of biometric information (an activity amount, a heart rate, a blood pressure, a body temperature, and the like) of the subject 2 during sleep (step SS5). When the device A has been removed from the subject 2 during the measurement of the biometric information, the device A interrupts the measurement of the biometric information and re-executes personal authentication in steps SS1 to SS3 in response to the device A that is being worn again on the subject 2 (step SS6). At this time, if the personal authentication is re-executed correctly, the measurement of the biometric information is resumed.

Then, if the measurement ends, the device B determines the propriety of driving of the vehicle 3 or the like by the subject 2 on the basis of, for example, the biometric information (an activity amount, a heart rate, a blood pressure, a body temperature, and the like) during sleep, a change in the biometric information, and/or a history of the biometric information 23, and transmits a propriety signal indicating the propriety determination to a device (not shown) of the vehicle 3 (a device that controls lock opening/closing and activation of the vehicle) (step SS7). A device (not shown) of the vehicle 3 controls the lock opening/closing of the vehicle 3 and the activation of the vehicle 3 on the basis of the propriety signal.

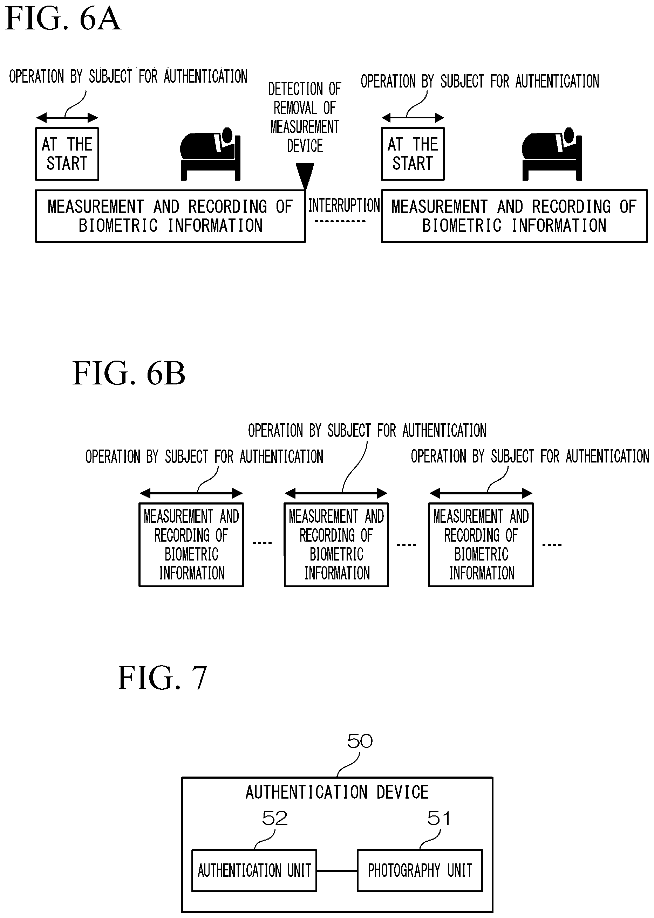

FIG. 6A is a conceptual diagram for describing an authentication operation of the biometric-information authentication system 1 according to the present example embodiment. FIG. 6B is a conceptual diagram for describing an authentication operation according to the background art. In the biometric-information authentication system 1 according to the present example embodiment, personal authentication is performed on the basis of the face of the subject 2 and the light emission pattern of the device A in a state in which the device A for measuring biometric information is worn on the subject 2, and personal authentication is performed in a state in which the device A is being worn again on the subject 2 if the device A has been removed.

As a result, if the device A is removed during the measurement of biometric information, personal authentication is executed in a state in which the device A is being worn again. Thus, it is possible to prevent spoofing in which the device A is transferred to another person to measure false biometric information after personal authentication is performed only when the measuring device A is activated.

In this way, because spoofing can be prevented, it is possible to ensure that the measured biometric information is the biometric information of a specific subject across the entire range of a specific period including a period in which the subject 2 is sleeping. Therefore, in the biometric-information authentication system 1 according to the present example embodiment, as shown in FIG. 6A, personal authentication in which the subject 2 is required to bear a burden or perform a special operation can be executed only for a partial period without being executed across the entire range of the specific period that also includes the period in which the subject 2 is sleeping, and the sleep of the subject 2 is not disturbed.

In contrast, for example, in Patent Documents 1 to 7 described above, as shown in FIG. 6B, an operation of the subject 2 is required with personal authentication across the entire range of a specific period in which biometric information is measured and the measured biometric information is recorded, and thus the sleep of the subject 2 is disturbed. In other words, it can be said that the above-mentioned Patent Documents 1 to 7 cannot cope with a case in which the biometric information is measured during a specific period that also includes the period in which the subject 2 is sleeping.

According to the above-described example embodiment, personal authentication is performed on the basis of the face of the subject 2 and the light emission pattern of the device A in a state in which the device A for measuring biometric information is worn on the subject 2 and personal authentication is performed in a state in which the device A is being worn again on the subject 2 if the device A has been removed. Therefore, it is possible to prevent spoofing in which the device A is transferred to another person to measure false biometric information after personal authentication is performed only when the measuring device A is activated.

Also, according to the above-described example embodiment, if the device A has been removed during the measurement of the biometric information, the personal authentication is performed again in a state in which the device A is being worn on the subject 2. Thus, personal authentication in which the subject 2 is required to bear a burden or perform a special operation can be executed only for a partial period (at the time of resumption) without being executed across the entire range of a specific period that also includes a period in which the subject 2 is sleeping, the biometric information of the subject 2 can be continuously measured in a state in which the sleep of the subject 2 is not disturbed.

Also, according to the above-described example embodiment, if the device A has been removed during the measurement of the biometric information, the personal authentication is performed again in a state in which the device A is being worn on the subject 2. Thus, it is possible to ensure that the measured biometric information is the biometric information of the specific subject across the entire range of the specific period including the period in which the subject 2 is sleeping.

FIG. 7 is a block diagram showing a minimum configuration of the authentication device according to the present example embodiment. An authentication device 50 according to the present example embodiment communicates with a measurement device that measures biometric information of a subject. Also, the authentication device 50 includes a photography unit 51 and an authentication unit 52. The photography unit 51 simultaneously photographs the subject and a predetermined light emission pattern in which light is emitted by the measurement device. The authentication unit 52 authenticates the subject and the measurement device on the basis of the subject and the predetermined light emission pattern photographed by the photography unit 51. Then, when a notification that the measurement device has been removed from the subject is received, the authentication device 50 re-executes authentication by the authentication unit 52.

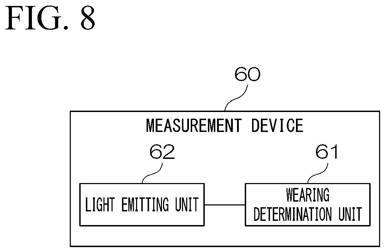

FIG. 8 is a block diagram showing a minimum configuration of the measurement device according to the present example embodiment. A measurement device 60 according to the present example embodiment is a measurement device that measures the biometric information of the subject. The measurement device 60 includes a wearing determination unit 61 and a light emitting unit 62. The wearing determination unit 61 determines whether or not the measurement device is being worn on the subject. If the wearing determination unit 61 determines that the measurement device is being worn on the subject, the light emitting unit 62 emits light in a predetermined light emission pattern. Then, if the wearing determination unit 61 determines that the measurement device has been removed from the subject during the measurement of the biometric information of the subject, the light emitting unit 62 of the measurement device 60 re-executes light emission in response to the measurement device that is being worn again on the subject.

It should be noted that the above-described processes (e.g., the processes shown in FIGS. 3 and 4) may be performed by recording a program for implementing the function of the biometric-information authentication system 1 in FIG. 1, the function of the authentication device 50 in FIG. 7, and the function of the measurement device 60 in FIG. 8 in a computer-readable recording medium and causing a computer system to read and execute the program recorded on the recording medium. Here, the recording medium refers to a magnetic disc, a magneto-optical disc, a compact disc (CD)-ROM, a digital versatile disc (DVD)-ROM, a semiconductor memory, or the like. Also, the program may be distributed to the computer via a communication circuit and the computer receiving the distributed program may execute the program. Also, the above-described program may be a program for implementing some of the above-described functions. Furthermore, the above-described program may be a program capable of implementing the above-described function in combination with a program already recorded in the computer system, that is, a so-called differential file (differential program). In addition, the biometric-information authentication system 1, the authentication device 50, and the measurement device 60 may be implemented using hardware such as a programmable logic device (PLD) or a field programmable gate array (FPGA).

Although example embodiments of the present invention have been described above with reference to the drawings, the present invention is not limited to the above-described example embodiments. Various changes, which can be understood by those skilled in the art, can be made in the configuration and details of the present invention within the scope of the present invention.

### INDUSTRIAL APPLICABILITY

The present invention relates to a biometric-information authentication system, a biometric-information authentication method, an authentication device, an authentication method, a measurement device, a measurement method, and a computer-readable recording medium having a program recorded thereon. According to the present invention, spoofing can be prevented.

### Description of Reference Signs

- 1: Biometric-information authentication system
- 2: Subject
- 3: Vehicle (external device)
- 10: Wireless communication unit
- 11: Light emission control unit
- 12: Display unit (light emitting unit)
- 13: Wearing determination unit
- 14: Biometric-information measurement unit (biometric-information measurement means)
- 20: Wireless communication unit
- 21: Storage unit
- 22: Authentication information
- 23: Biometric information
- 24: Photography unit
- 25: Display unit
- 26: Personal authentication unit
- 27: Propriety determination unit (propriety determination means)
- 50: Authentication device
- 51: Photography unit (photography means)
- 52: Authentication unit (authentication means)
- 60: Measurement device
- 61: Wearing determination unit (wearing determination means)
- 62: Light emitting unit (light emitting means)
- A: Device (measurement device)
- B: Device

## Claims

1. A biometric-information authentication system comprising:
a measurement device (A) that measures biometric information of a subject (2); and
an authentication device (B) that communicates with the measurement device (A),
wherein the measurement device (A) comprises:
a wearing determination means (13) that determines whether or not the measurement device (A) is being worn on the subject (2); and
a light emitting means (12) that emits light in a predetermined light emission pattern when the wearing determination means (13) determines that the measurement device (A) is being worn on the subject (2),
the authentication device (B) comprises:
a photography means (24) that photographs the subject (2) and the predetermined light emission pattern simultaneously; and
an authentication means (26) that authenticates the subject (2) and the measurement device (A) on the basis of the subject (2) and the predetermined light emission pattern that have been photographed by the photography means (24),
the measurement device (A) re-executes light emission by the light emitting means (12) in response to the measurement device (A) that is being worn again on the subject (2) when the wearing determination means (13) determines that the measurement device (A) has been removed from the subject (2) during measurement of the biometric information of the subject (2), and
the authentication device (B) re-executes authentication by the authentication means (26) when a notification that the measurement device (A) has been removed from the subject (2) has been received from the measurement device (A),
**characterized in that**
the measurement device (A) further comprises a biometric-information measurement means (14) that starts the measurement of the biometric information of the subject (2) after the authentication device (B) has authenticated the subject (2) and the measurement device (A).

2. The biometric-information authentication system according to claim 1, wherein the authentication device (B) further comprises a propriety determination means (27) that determines propriety of an operation of an external device (3) on the basis of the biometric information measured by the measurement device (A).

3. The biometric-information authentication system according to claim 1, wherein the authentication device (B) further comprises a propriety determination means (27) that determines propriety of lock opening/closing of a vehicle (3) on the basis of the biometric information measured by the measurement device (A).

4. The biometric-information authentication system according to claim 1, wherein the authentication device (B) further comprises a propriety determination means (27) that, on the basis of the biometric information measured by the measurement device (A), determines whether or not the subject (2) has obtained sleep and determines propriety of driving of a vehicle (3) by the subject (2).

5. The biometric-information authentication system according to claim 1, wherein the light emission pattern and face information of the subject (2) are associated with each other.

6. A biometric-information authentication method comprising:
determining, by a measurement device (A) that measures biometric information of a subject (2), whether or not the measurement device (A) is being worn on the subject (2);
causing a light emitting means (12) of the measurement device (A) to emit light in a predetermined light emission pattern when it is determined that the measurement device (A) is being worn on the subject (2);
photographing, by an authentication device (B) that communicates with the measurement device (A), the subject (2) and the predetermined light emission pattern simultaneously;
authenticating, by the authentication device (B), the subject (2) and the measurement device (A) on the basis of the subject (2) and the predetermined light emission pattern that have been photographed;
re-executing, by the measurement device (A), light emission by the light emitting means (12) in response to the measurement device (A) that is being worn again on the subject (2) when it is determined that the measurement device (A) has been removed from the subject (2) during measurement of the biometric information of the subject (2); and
re-executing, by the authentication device (B), authentication when a notification that the measurement device (A) has been removed from the subject (2) has been received from the measurement device (A),
**characterized in that** the method further comprises:
starting, by the measurement device (A), the measurement of the biometric information of the subject (2) after the authentication device (B) has authenticated the subject (2) and the measurement device (A).

7. A measurement device (A) for measuring biometric information of a subject (2), the measurement device (A) comprising:
a wearing determination means (13) that determines whether or not the measurement device (A) is being worn on the subject (2); and
a light emitting means (12) that emits light in a predetermined light emission pattern when the wearing determination means (13) determines that the measurement device (A) is being worn on the subject (2),
wherein the measurement device (A) re-executes light emission by the light emitting means (12) in response to the measurement device (A) that is being worn again on the subject (2) when the wearing determination means (13) determines that the measurement device (A) has been removed from the subject (2) during measurement of the biometric information of the subject (2),
**characterized in that**
the measurement device (A) further comprises a biometric-information measurement means (14) that starts the measurement of the biometric information of the subject (2) after receiving an indication from an authentication device (B) that the subject (2) and the measurement device (A) have been authenticated.

8. A measurement method in a measurement device (A) for measuring biometric information of a subject (2), the measurement method comprising:
determining whether or not the measurement device (A) is being worn on the subject (2);
causing a light emitting means (12) of the measurement device (A) to emit light in a predetermined light emission pattern when it is determined that the measurement device (A) is being worn on the subject (2); and
re-executing light emission by the light emitting means (12) in response to the measurement device (A) that is being worn again on the subject (2) when it is determined that the measurement device (A) has been removed from the subject during measurement of the biometric information of the subject (2),
**characterized in that** the method further comprises:
starting the measurement of the biometric information of the subject (2) after receiving an indication from an authentication device (B) that the subject (2) and the measurement device (A) have been authenticated.

9. A program that causes a measurement device (A) for measuring biometric information of a subject (2) to execute:
a step of determining whether or not the measurement device (A) is being worn on the subject (2);
a step of causing a light emitting means (12) of the measurement device (A) to emit light in a predetermined light emission pattern when it is determined that the measurement device (A) is being worn on the subject (2); and
a step of re-executing light emission by the light emitting means (12) in response to the measurement device (A) that is being worn again on the subject (2) when it is determined that the measurement device (A) has been removed from the subject (2) during measurement of the biometric information of the subject (2),
**characterized in that** the program further causes the measurement device (A) to execute:
a step of starting the measurement of the biometric information of the subject (2) after receiving an indication from an authentication device (B) that the subject (2) and the measurement device (A) have been authenticated.

## Patentansprüche

1. Authentifizierungssystem für biometrische Informationen, das aufweist:
eine Messvorrichtung (A), die biometrische Informationen einer Person (2) misst; und
eine Authentifizierungsvorrichtung (B), die mit der Messvorrichtung (A) kommuniziert, wobei die Messvorrichtung (A) aufweist:
eine Tragebestimmungseinrichtung (13), die bestimmt, ob die Messvorrichtung (A) von der Person (2) getragen wird oder nicht; und
eine lichtemittierende Einrichtung (12), die Licht in einem vorbestimmten Lichtemissionsmuster emittiert, wenn die Tragebestimmungseinrichtung (13) bestimmt, dass die Messvorrichtung (A) an der Person (2) getragen wird,
wobei die Authentifizierungsvorrichtung (B) aufweist:
eine Fotoeinrichtung (24), die die Person (2) und das vorbestimmte Lichtemissionsmuster gleichzeitig fotografiert; und
eine Authentifizierungseinrichtung (26), die die Person (2) und die Messvorrichtung (A) auf der Grundlage der Person (2) und des vorbestimmten Lichtemissionsmusters authentifiziert, die von der Fotoeinrichtung (24) fotografiert worden sind,
wobei die Messvorrichtung (A) die Lichtemission durch die lichtemittierende Einrichtung (12) als Reaktion auf die Messvorrichtung (A), die wieder an der Person (2) getragen wird, erneut ausführt, wenn die Tragebestimmungseinrichtung (13) feststellt, dass die Messvorrichtung (A) während der Messung der biometrischen Informationen der Person (2) von der Person (2) entfernt worden ist, und
die Authentifizierungsvorrichtung (B) die Authentifizierung durch die Authentifizierungseinrichtung (26) erneut ausführt, wenn eine Benachrichtigung von der Messvorrichtung (A) empfangen worden ist, dass die Messvorrichtung (A) von der Person (2) entfernt worden ist,
**dadurch gekennzeichnet, dass**
die Messvorrichtung (A) ferner eine Messeinrichtung (14) biometrischer Informationen aufweist, die die Messung der biometrischen Informationen der Person (2) beginnt, nachdem die Authentifizierungsvorrichtung (B) die Person (2) und die Messvorrichtung (A) authentifiziert hat.

2. Authentifizierungssystem für biometrische Informationen nach Anspruch 1, wobei die Authentifizierungsvorrichtung (B) ferner eine Korrektheitsbestimmungseinrichtung (27) aufweist, die auf der Grundlage der von der Messvorrichtung (A) gemessenen biometrischen Informationen die Korrektheit eines Betriebs einer externen Vorrichtung (3) bestimmt.

3. Authentifizierungssystem für biometrische Informationen nach Anspruch 1, wobei die Authentifizierungsvorrichtung (B) ferner eine Korrektheitsbestimmungseinrichtung (27) aufweist, die auf der Grundlage der von der Messvorrichtung (A) gemessenen biometrischen Informationen die Korrektheit des Öffnens/Schließens eines Fahrzeugs (3) bestimmt.

4. Authentifizierungssystem für biometrische Informationen nach Anspruch 1, wobei die Authentifizierungsvorrichtung (B) ferner eine Korrektheitsbestimmungseinrichtung (27) aufweist, die auf der Grundlage der von der Messvorrichtung (A) gemessenen biometrischen Informationen bestimmt, ob die Person (2) Schlaf erhalten hat oder nicht, und die Korrektheit des Fahrens eines Fahrzeugs (3) durch die Person (2) bestimmt.

5. Authentifizierungssystem für biometrische Informationen nach Anspruch 1, wobei das Lichtemissionsmuster und Gesichtsinformation der Person (2) miteinander verbunden sind.

6. Authentifizierungsverfahren für biometrische Informationen, das aufweist:
Bestimmen, durch eine Messvorrichtung (A), die biometrische Informationen einer Person (2) misst, ob die Messvorrichtung (A) an der Person (2) getragen wird oder nicht;
Veranlassen einer lichtemittierenden Einrichtung (12) der Messvorrichtung (A), Licht in einem vorbestimmten Lichtemissionsmuster zu emittieren, wenn bestimmt wird, dass die Messvorrichtung (A) an der Person (2) getragen wird;
gleichzeitiges Fotografieren, durch eine Authentifizierungsvorrichtung (B), die mit der Messvorrichtung (A) kommuniziert, der Person (2) und des vorbestimmten Lichtemissionsmusters;
Authentifizieren, durch die Authentifizierungsvorrichtung (B), der Person (2) und der Messvorrichtung (A) auf der Grundlage der Person (2) und des vorbestimmten Lichtemissionsmusters, die fotografiert worden sind;
erneutes Ausführen, durch die Messvorrichtung (A), der Lichtemission durch die lichtemittierende Einrichtung (12) als Reaktion auf die Messvorrichtung (A), die wieder an der Person (2) getragen wird, wenn bestimmt wird, dass die Messvorrichtung (A) während der Messung der biometrischen Informationen der Person (2) von der Person (2) entfernt worden ist; und
erneutes Ausführen, durch die Authentifizierungsvorrichtung (B), des Authentifizierens wenn eine Benachrichtigung von der Messvorrichtung (A) empfangen worden ist, dass die Messvorrichtung (A) von der Person (2) entfernt worden ist,
**dadurch gekennzeichnet, dass** das Verfahren ferner aufweist:
Beginnen, durch die Messvorrichtung (A), der Messung der biometrischen Information der Person (2), nachdem die Authentifizierungsvorrichtung (B) die Person (2) und die Messvorrichtung (A) authentifiziert hat.

7. Messvorrichtung (A) zum Messen biometrischer Informationen einer Person (2), wobei die Messvorrichtung (A) aufweist:
eine Tragebestimmungseinrichtung (13), die bestimmt, ob die Messvorrichtung (A) von der Person (2) getragen wird oder nicht; und
eine lichtemittierende Einrichtung (12), die Licht in einem vorbestimmten Lichtemissionsmuster emittiert, wenn die Tragebestimmungseinrichtung (13) bestimmt, dass die Messvorrichtung (A) an der Person (2) getragen wird,
wobei die Messvorrichtung (A) die Lichtemission durch die lichtemittierende Einrichtung (12) als Reaktion auf die Messvorrichtung (A), die wieder an der Person (2) getragen wird, erneut ausführt, wenn die Tragebestimmungseinrichtung (13) bestimmt, dass die Messvorrichtung (A) während der Messung der biometrischen Informationen der Person (2) von der Person (2) entfernt worden ist,
**dadurch gekennzeichnet, dass**
die Messvorrichtung (A) ferner eine Messeinrichtung (14) biometrischer Informationen aufweist, die die Messung der biometrischen Informationen der Person (2) beginnt, nachdem sie eine Anzeige von einer Authentifizierungsvorrichtung (B) empfangen hat, dass die Person (2) und die Messvorrichtung (A) authentifiziert worden sind.

8. Messverfahren in einer Messvorrichtung (A) zum Messen biometrischer Informationen einer Person (2), wobei das Messverfahren aufweist:
Bestimmen, ob die Messvorrichtung (A) von der Person (2) getragen wird oder nicht;
Veranlassen einer lichtemittierenden Einrichtung (12) der Messvorrichtung (A), Licht in einem vorbestimmten Lichtemissionsmuster zu emittieren, wenn bestimmt wird, dass die Messvorrichtung (A) an der Person (2) getragen wird; und
erneutes Ausführen der Lichtemission durch die lichtemittierende Einrichtung (12) als Reaktion auf die Messvorrichtung (A), die wieder an der Person (2) getragen wird, wenn bestimmt wird, dass die Messvorrichtung (A) während der Messung der biometrischen Informationen der Person (2) von der Person entfernt worden ist,
**dadurch gekennzeichnet, dass** das Verfahren ferner aufweist:
Beginnen der Messung der biometrischen Information der Person (2) nach dem Empfangen einer Anzeige von einer Authentifizierungsvorrichtung (B), dass die Person (2) und die Messvorrichtung (A) authentifiziert worden sind.

9. Programm, das eine Messvorrichtung (A) zum Messen biometrischer Informationen einer Person (2) veranlasst, auszuführen:
einen Schritt des Bestimmens, ob die Messvorrichtung (A) von der Person (2) getragen wird oder nicht;
einen Schritt des Veranlassens einer lichtemittierenden Einrichtung (12) der Messvorrichtung (A), Licht in einem vorbestimmten Lichtemissionsmuster zu emittieren, wenn bestimmt wird, dass die Messvorrichtung (A) an der Person (2) getragen wird; und
einen Schritt des erneuten Ausführens der Lichtemission durch die lichtemittierende Einrichtung (12) als Reaktion auf die Messvorrichtung (A), die wieder an der Person (2) getragen wird, wenn bestimmt wird, dass die Messvorrichtung (A) während der Messung der biometrischen Informationen der Person (2) von der Person (2) entfernt worden ist, **dadurch gekennzeichnet, dass** das Programm ferner die Messvorrichtung (A) veranlasst, auszuführen:
einen Schritt des Beginnens der Messung der biometrischen Informationen der Person (2) nach dem Empfang einer Anzeige von einer Authentifizierungsvorrichtung (B), dass die Person (2) und die Messvorrichtung (A) authentifiziert worden sind.

## Revendications

1. Système d'authentification par informations biométriques, comprenant :
un dispositif de mesure (A) mesurant des informations biométriques d'un sujet (2) ; et
un dispositif d'authentification (B) communiquant avec le dispositif de mesure (A),
où le dispositif de mesure (A) comprend :
un moyen de détermination de port (13) qui détermine si le dispositif de mesure (A) est porté ou non par le sujet (2) ; et
un moyen d'émission lumineuse (12) émettant de la lumière suivant un motif d'émission lumineuse prédéterminé si le moyen de détermination de port (13) détermine que le dispositif de mesure (A) est porté par le sujet (2),
où le dispositif d'authentification (B) comprend :
un moyen photographique (24) photographiant simultanément le sujet (2) et le motif d'émission lumineuse prédéterminé ; et
un moyen d'authentification (26) authentifiant le sujet (2) et le dispositif de mesure (A) sur la base du sujet (2) et du motif d'émission lumineuse prédéterminé ayant été photographiés par le moyen photographique (24),
où le dispositif de mesure (A) déclenche une nouvelle émission de lumière par le moyen d'émission lumineuse (12) en réaction à un nouveau port du dispositif de mesure (A) par le sujet (2) si le moyen de détermination de port (13) détermine que le dispositif de mesure (A) a été retiré du sujet (2) pendant la mesure des informations biométriques du sujet (2), et
où le dispositif d'authentification (B) déclenche une nouvelle authentification par le moyen d'authentification (26) si une notification indiquant que le dispositif de mesure (A) a été retiré du sujet (2) a été reçue du dispositif de mesure (A),
**caractérisé en ce que**
le dispositif de mesure (A) comprend en outre un moyen de mesure d'informations biométriques (14) démarrant la mesure des informations biométriques du sujet (2) après authentification du sujet (2) et du dispositif de mesure (A) par le dispositif d'authentification (B).

2. Système d'authentification par informations biométriques selon la revendication 1, où le dispositif d'authentification (B) comprend en outre un moyen de détermination de pertinence (27) déterminant la pertinence d'un actionnement d'un dispositif externe (3) sur la base des informations biométriques mesurées par le dispositif de mesure (A).

3. Système d'authentification par informations biométriques selon la revendication 1, où le dispositif d'authentification (B) comprend en outre un moyen de détermination de pertinence (27) déterminant la pertinence d'une ouverture/fermeture de serrure d'un véhicule (3) sur la base des informations biométriques mesurées par le dispositif de mesure (A).

4. Système d'authentification par informations biométriques selon la revendication 1, où le dispositif d'authentification (B) comprend en outre un moyen de détermination de pertinence (27) qui, sur la base des informations biométriques mesurées par le dispositif de mesure (A), détermine si le sujet (2) a dormi et détermine la pertinence de la conduite d'un véhicule (3) par le sujet (2).

5. Système d'authentification par informations biométriques selon la revendication 1, où le motif d'émission lumineuse et des informations sur le visage du sujet (2) sont associés entre eux.

6. Procédé d'authentification par informations biométriques, comprenant :
la détermination, par un dispositif de mesure (A) mesurant des informations biométriques d'un sujet (2), si le dispositif de mesure (A) est porté ou non par le sujet (2) ;
le déclenchement d'une émission de lumière par un moyen d'émission lumineuse (12) du dispositif de mesure (A), suivant un motif d'émission lumineuse prédéterminé, s'il est déterminé que le dispositif de mesure (A) est porté par le sujet (2) ;
la photographie simultanée, par un dispositif d'authentification (B) communiquant avec le dispositif de mesure (A), du sujet (2) et du motif d'émission lumineuse prédéterminé ;
l'authentification, par le dispositif d'authentification (B), du sujet (2) et du dispositif de mesure (A) sur la base du sujet (2) et du motif d'émission lumineuse prédéterminé ayant été photographiés ;
le déclenchement, par le dispositif de mesure (A), d'une nouvelle émission de lumière par le moyen d' émission lumineuse (12) en réaction à un nouveau port du dispositif de mesure (A) par le sujet (2) s'il est déterminé que le dispositif de mesure (A) a été retiré du sujet (2) pendant la mesure des informations biométriques du sujet (2) ; et
la réexécution d'une authentification par le dispositif d'authentification (B), si une notification indiquant que le dispositif de mesure (A) a été retiré du sujet (2) a été reçue du dispositif de mesure (A),
**caractérisé en ce que** ledit procédé comprend en outre :
le démarrage, par le dispositif de mesure (A), de la mesure des informations biométriques du sujet (2) après authentification du sujet (2) et du dispositif de mesure (A) par le dispositif d'authentification (B).

7. Dispositif de mesure (A) pour mesurer des informations biométriques d'un sujet (2), ledit dispositif de mesure (A) comprenant :
un moyen de détermination de port (13) déterminant si le dispositif de mesure (A) est porté ou non par le sujet (2) ; et
un moyen d'émission lumineuse (12) émettant de la lumière suivant un motif d'émission lumineuse prédéterminé si le moyen de détermination de port (13) détermine que le dispositif de mesure (A) est porté par le sujet (2),
où le dispositif de mesure (A) déclenche une nouvelle émission de lumière par le moyen d'émission lumineuse (12) en réaction à un nouveau port du dispositif de mesure (A) par le sujet (2) si le moyen de détermination de port (13) détermine que le dispositif de mesure (A) a été retiré du sujet (2) pendant la mesure des informations biométriques du sujet (2), **caractérisé en ce que**
ledit dispositif de mesure (A) comprend en outre un moyen de mesure d'informations biométriques (14) démarrant la mesure des informations biométriques du sujet (2) après réception d'une indication d'un dispositif d'authentification (B) selon laquelle le sujet (2) et le dispositif de mesure (A) ont été authentifiés.

8. Procédé de mesure dans un dispositif de mesure (A) pour mesurer des informations biométriques d'un sujet (2), ledit procédé de mesure comprenant :
la détermination si le dispositif de mesure (A) est porté ou non par le sujet (2) ;
le déclenchement d'une émission de lumière par un moyen d'émission lumineuse (12) du dispositif de mesure (A), suivant un motif d'émission lumineuse prédéterminé, s'il est déterminé que le dispositif de mesure (A) est porté par le sujet (2) ; et
le déclenchement, par le moyen d'émission lumineuse (12), d'une nouvelle émission de lumière par le moyen d'émission lumineuse (12) en réaction à un nouveau port du dispositif de mesure (A) par le sujet (2) s'il est déterminé que le dispositif de mesure (A) a été retiré du sujet (2) pendant la mesure des informations biométriques du sujet (2),
**caractérisé en ce que** ledit procédé comprend en outre :
le démarrage de la mesure des informations biométriques du sujet (2) après réception d'une indication d'un dispositif d'authentification (B) selon laquelle le sujet (2) et le dispositif de mesure (A) ont été authentifiés.

9. Programme, déclenchant l'exécution par un dispositif de mesure (A) d'informations biométriques d'un sujet (2) :
d'une étape de détermination si le dispositif de mesure (A) est porté ou non par le sujet (2) ;
d'une étape de déclenchement d'une émission de lumière par un moyen d'émission lumineuse (12) du dispositif de mesure (A), suivant un motif d'émission lumineuse prédéterminé, s'il est déterminé que le dispositif de mesure (A) est porté par le sujet (2) ; et
d'une étape de déclenchement, par le dispositif de mesure (A), d'une nouvelle émission de lumière par le moyen d'émission lumineuse (12) en réaction à un nouveau port du dispositif de mesure (A) par le sujet (2) s'il est déterminé que le dispositif de mesure (A) a été retiré du sujet (2) pendant la mesure des informations biométriques du sujet (2),
**caractérisé en ce que** ledit programme déclenche en outre l'exécution par le dispositif de mesure (A) :
d'une étape de démarrage de la mesure des informations biométriques du sujet (2) après réception d'une indication d'un dispositif d'authentification (B) selon laquelle le sujet (2) et le dispositif de mesure (A) ont été authentifiés.
